(19) **Europäisches Patentamt / European Patent Office / Office européen des brevets**

(11) **EP 3 751 577 A1**

(12) # EUROPEAN PATENT APPLICATION

(43) Date of publication:
16.12.2020  Bulletin 2020/51

(51) Int Cl.:
$G16H\ 20/30$ (2018.01)      $G16H\ 50/30$ (2018.01)

(21) Application number: **19180212.3**

(22) Date of filing: **14.06.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Elmedix NV**
**3001 Leuven (BE)**

(72) Inventor: **Van den Bossche, Johan**
**B-3210 Linden (BE)**

(74) Representative: **IPLodge bv**
**Technologielaan 9**
**3001 Heverlee (BE)**

(54) **SIMULATION OF TEMPERATURES IN THE BODY**

(57)    The invention pertains to a method (100) for simulating temperatures in the body when in a controllable environment. The method comprises receiving (101) a property of the body obtained by measurements, calculating (102) a body-specific parameter of a bioheat model based on the property, obtaining (103) at least one environment-specific parameter, and calculating (104) the temperatures in the body by solving the model taking the body-specific and environment-specific parameters into account. The equivalent thermal circuit (50) of the model comprises a blood compartment (51) and a plurality of anatomical segments (52, 53, 54, 55), each comprising a plurality of compartments (56, 57, 58). The interior compartments of a trunk segment (54) comprises organ compartments (561, 562) representative of different internal organs, which are connected in parallel in the equivalent thermal circuit. The invention also relates to a device, system and computer-program product.

FIG 1

**Description**

**Technical Field**

The present invention relates to the field of body temperature manipulation of the human or animal body, and particularly to the simulation of temperatures and/or heat flows in the body when subjected to controlled heat and/or cooling conditions, such as when the body is encapsulated in a controlled microclimate. The present invention specifically relates to a method for predicting and/or simulating temperatures in the body when the body is exposed to a controllable environment, e.g. predicting and/or simulating internal temperatures (e.g. of organs) as function of controllable parameters of the environment. The invention also relates to devices, computer-program products and systems directed to this purpose.

**Background Art**

Various conditions may require a detailed simulation of temperatures and/or heat flows inside the human or animal body. For example, in hyperthermia treatment, hyperthermia can be deliberately induced by drugs and/or medical devices as a primary or adjuvant treatment of cancer. For example, whole-body hyperthermia is a promising cancer treatment, in which the body is heated to, for example, 41.5 °C to overheat cancerous cells. This approach may be particularly useful for treating cancers that are widely distributed in the body. However, this type of treatment poses substantial challenges in determining the amount of energy that can be safely delivered to the body in the form of heat to induce whole-body hyperthermia, and, particularly, in the process of accurately modeling and/or correctly predicting temperatures in the body under treatment.

For example, pancreas cancer is a particularly aggressive cancer that easily metastasizes, for which whole-body hyperthermia could offer a promising treatment. However, because of the risk of local overheating in the body, heat management has to be performed with care. For example, the liver has a high metabolic rate, and may therefore be prone to overheating when the whole body is brought to an elevated temperature. Such overheating could lead to acute liver failure. Therefore, a need in the art exists for accurate thermal simulation and modeling of temperatures in the body.

While simulation of hyperthermia treatment is considered as an important application of the present invention, embodiments are not necessarily limited thereto. Furthermore, not only hyperthermia may be used in a therapeutic setting, but controlled hypothermia is also known in the art as an active treatment, for example during recovery after a period of stopped blood flow to the brain, e.g. in perinatal hypoxia-ischemia, hypoxic ischemic encephalopathy or birth asphyxia, or after resuscitation following cardiac arrest. Therapeutic hypothermia may prevent brain injury by decreasing the oxygen demand in the brain, reducing the production of specific neurotransmitters and/or by reducing free radicals.

In the present disclosure, targeted temperature management refers to applications in which the human or animal body is subjected to controlled conditions to achieve and maintain a specific body temperature (e.g. a predetermined constant body temperature for a predetermined duration of time, or a body temperature being a predetermined function of time over a predetermined time period), e.g. which may be above or below the normal core body temperature. Other examples in which an accurate simulation of temperatures and/or heat flows in the animal, e.g. mammal, or human body may be advantageous or desirable include the controlled heating and/or cooling of the body after severe heat stroke or after severe hypothermia. Possible applications in induced torpor might also be considered conceivable.

Applications are also not necessarily limited to medical applications, but may include applications such as the control of personal protective equipment encapsulating the wearer in a microclimate, such as for hazard protection of chemical agents, gases, fire, explosion, or such as diving suits, hot or cold environment protection suits or space suits, i.e. such suits in which the internal thermal microclimate is actively controlled.

The bioheat generation and transfer within a human (or animal) body is a complex combination of processes, including internal heat transfer by conduction, e.g. through tissues, internal heat transfer by convection of bodily fluids, e.g. due to blood perfusion, heat generation due to metabolic processes, respiratory heat losses, heat losses facilitated by perspiration and external heating and/or cooling by convection, radiation and/or conduction. While a need exists in for methods and devices to accurate simulate and model temperatures and thermal processes in the body, a fine-grained modeling of each of these effects, e.g. at the microscopic level, throughout the human body is extremely difficult, if not outright infeasible. Since low tolerance margins may be imposed, e.g. particularly in the case of whole-body hyperthermia treatment modeling, a simulation and modeling approach preferably should also be adjustable to subject-specific parameters, to account for inter-subject variability.

Paulides et al., "Simulation techniques in hyperthermia treatment planning;" in International journal of hyperthermia 29(4), pp. 346-357, discussed simulation tools and techniques developed for personalized hyperthermia treatment planning of local hyperthermia treatment by locally heating tissues using waves. These planning approaches comprise a numerical segmentation of volumetric image data obtained from the patient, a simulation of (part of) the patient in a heat delivery setup, e.g. an ultrasound or electromagnetic irradiation system, and a simulation of the resulting temperature distribution in the segmented tissues. However, Paulides did not consider the modeling of a whole-body hyperthermia treatment, which clearly poses different challenges in terms of safeguarding organs at risk from overheating due to a combination of metabolic heat generation and externally applied heat. Furthermore, Paulides acknowledges the difficulties that are encountered when attempting to construct a fine-grained thermal model of the body, due to, for example, variation in thermal properties of tissues, patient-specific thermoregulation responses and the small-scale heterogenous distribution of thermally significant blood vessels, particularly as influenced by tumor growth.

The document CN102012973A describes a human body thermal simulation modeling method that is suitable for a complex space environment. The method comprises dividing a human body model into a human body hyperpyrexia core area, a blood perfusion area and an equivalent tissue heat transfer area from inside to outside, and solving the temperature distribution of the core area, the blood perfusion area and the heat transfer area by adopting an unsteady state heat transfer equation. By introducing the self-thermoregulation behavior of the human body, the temperature distribution of a human body surface is obtained. However, this document is concerned with the analysis of a comfort level as function of environmental parameters, and not with health risks associated with overheating of the body and/or specific organs of the body.

Xiang et al, "Comprehensive evaluation on the heating capacities of four typical whole body hyperthermia strategies via compartmental model," in International Journal of Heat and Mass Transfer 51(23-34), pp. 5486-5496, describes a compartmental bioheat model for evaluating whole-body hyperthermia protocols. In this compartmental model, the human body is approximated by twelve body segments and a blood compartment. Each body segment is modeled as a multi-layered cylinder, except the head, which is modeled as a multi-layered sphere. In such a compartmental model, spatial variations of variables and properties within a compartment are ignored and simplified by a single-valued representation of the variable or property. The four concentric layers of these cylinders (resp. sphere) act as compartments of the model, which have variables and properties associated therewith, for example (and particularly noteworthy) a temperature.

## Summary of the invention

It is an objective of the present invention to good and efficient means and methods for simulating and/or predicting internal temperatures of the human or animal body when in a controllable environment, e.g. taking control parameters of the controllable environment into account.

Embodiments of the present invention have the advantage that a simple and efficient model, e.g. a computationally efficient model, is provided for performing subject-specific simulations and/or predictions of internal temperatures, e.g. of a temperature spatial distribution, in the body.

Embodiments of the present invention have the advantage that one or more simulations can be performed to determine control parameters for a controllable environment, such that, by exposing a human or animal body to the controllable environment controlled according to said control parameters, a set of predetermined constraints on a plurality of different internal temperatures, e.g. organ temperatures and/or blood temperature, is satisfied.

Embodiments of the present invention have the advantage that a subject-specific, i.e. body-specific, simulation can be performed to accurately simulate internal temperatures in the body.

Embodiments of the present invention have the advantage that a whole-body hyperthermia treatment can be planned in a simple, efficient and/or effective manner.

Embodiments of the present invention have the advantage that a whole-body hyperthermia treatment can be planned such as to ensure or improve safety of the patient and efficacy of the treatment.

Embodiments of the present invention have the advantage that a whole-body hyperthermia treatment can be controlled in real-time (or near real-time) taking estimates of internal body temperatures obtained by simulation into account.

Embodiments of the present invention have the advantage that a good model is provided for simulating both specific organ temperatures and temperatures of larger body regions, while requiring relatively few computing resources, e.g. memory, processing time and/or processing power. For example, it has been found that a steady-state simulation model can be encoded in the form of a spreadsheet, and can be solved by standard spreadsheet solver functionality. This also provides, advantageously, a high degree of portability.

In a first aspect, the present invention relates to a computer-implemented method for predicting and/or simulating a plurality of temperatures in a human or animal body when said body is in a predetermined environment, e.g. a controllable environment or a controlled environment, e.g. an environment subject to (thermal) control by adjustable parameters. The predetermined environment comprises controllable heating and/or cooling means for bringing a core body temperature of said body to a predetermined target temperature and/or maintaining the core body temperature at said predetermined target temperature. The method comprises receiving as input at least one property of said body obtained by measurements of the body and calculating at least one body-specific parameter of a bioheat model based on the at least one property of the body. The method comprises obtaining at least one environment-specific parameter that is indicative of at least one thermal property (e.g. at least one controllable thermal property) of the predetermined environment and/or of at least one property of a thermal exchange between said body and said predetermined environment. The method comprises calculating said temperatures in the body by numerically solving said bioheat model, thereby taking said body-specific parameters and said environment-specific parameters into account.

The bioheat model comprises a plurality of equations representative of an equivalent thermal circuit for modeling heat flows in the body and between the body and the predetermined environment. The equivalent thermal circuit comprises a blood compartment and a plurality of anatomical segments, the plurality of anatomical segments comprising at least a lower extremity segment, an upper extremity segment, a trunk segment and a head segment. Each of the anatomical segments comprises a plurality of compartments. This plurality of compartments comprises at least one interior compartment representative of a core and/or bone part of the anatomical segment, at least one intermediate compartment representative of a muscle and/or fat part of the anatomical segment and an exterior compartment representative of a skin part of the anatomical segment.

The at least one interior compartment of the at least one trunk segment comprises a plurality of organ compartments representative of respectively at least two internal organs. The at least two organ compartments are connected in parallel in said equivalent thermal circuit.

In a method in accordance with embodiments of the present invention, the temperatures may comprise at least a temperature of each of said organ compartments and a temperature of said blood compartment, e.g. may comprise a temperature corresponding to each compartment of the model.

In a method in accordance with embodiments of the present invention, the obtaining of the at least one environment-specific parameter may comprise obtaining a plurality of different values of the or each environment-specific parameter. The step of calculating the temperatures may be performed for each of said different values to determine changes in said temperatures with respect to said at least one environment-specific parameter.

In a method in accordance with embodiments of the present invention, said obtaining of the at least one environment-specific parameter may comprise adjusting a previous value of said plurality of different values for which said temperatures were calculated to determine a next value in said plurality of different values for which said temperatures are (to be) calculated, in order to optimize one or more of said temperatures toward one or more corresponding target temperatures and/or to meet one or more constraints on said temperatures.

A method in accordance with embodiments of the present invention may comprise receiving, as input, sensor data from one or more temperature sensors on or in the body, and taking said sensor data into account when numerically solving said bioheat model.

In a method in accordance with embodiments of the present invention, the at least one trunk segment may comprise an abdomen segment and a thorax segment. The plurality of organ compartments may be interior compartments of the abdomen segment.

In a method in accordance with embodiments of the present invention, the at least one property of the body may comprise one or more properties of the body that are indicative of a body weight, a body volume, body and/or body part dimensions, and/or a body composition.

In a method in accordance with embodiments of the present invention, said calculating of the at least one body-specific parameter may comprise determining values representative of thermal conduction between at least some of the compartments of said model based on said at least one property of the body.

In a method in accordance with embodiments of the present invention, the at least one environment-specific parameter may comprise at least one value representative of a convection parameter and/or a temperature of an air flow to which the body is exposed in said environment.

In a method in accordance with embodiments of the present invention, the at least one environment-specific parameter may comprise at least one value representative of a temperature of surroundings of the body in said environment for use in calculations of radiative heat transport.

In a method in accordance with embodiments of the present invention, the at least one environment-specific parameter may comprise at least one value indicative of a temperature of an actively heated and/or cooled surface contacting at least part of the body in said environment.

In a method in accordance with embodiments of the present invention, the at least one environment-specific parameter may comprise at least one value indicative of a temperature and/or a humidity of air inspired by the body in said environment.

In a method in accordance with embodiments of the present invention, the at least one environment-specific parameter may comprise at least one value indicative of a controllable thermal property of a heater and/or cooler for heating and/or cooling blood of the body in said environment.

A method in accordance with embodiments of the present invention may comprise calculating a value representative of a rate of convective heat exchange for each exterior compartment based on the at least one value representative of the convection parameter and/or the temperature of the air flow to which the body is exposed in said environment.

A method in accordance with embodiments of the present invention may comprise calculating a value representative of a rate of radiative heat exchange for each exterior compartment based on the at least one value representative of the temperature of the surroundings in the environment.

A method in accordance with embodiments of the present invention may comprise calculating a value representative of a rate of conductive heat exchange for an exterior compartment based on the at least one value indicative of the temperature of the actively heated and/or cooled surface.

A method in accordance with embodiments of the present invention may comprise calculating a value representative of a rate of heat exchange by respiration based on the at least one value indicative of the temperature and/or the humidity of the inspired air.

In a method in accordance with embodiments of the present invention, calculating any of the values representative of the rate of convective and/or radiative and/or conductive heat exchange may take a surface area into account, in which this surface area is obtained or calculated from the at least one property of the body received as input.

In a method in accordance with embodiments of the present invention, said at least one interior compartment of said trunk segment that comprises the at least two organ compartments may also comprise a remainder compartment representative of the remainder of the interior part of said trunk segment. The remainder compartment and the at least two organ compartments may be connected in parallel in the equivalent thermal circuit.

Embodiments of the present invention may also relate to a method that comprises measuring at least one property of a (specific) human or animal body, providing the at least one property of the body as input to a computer-implemented method in accordance with embodiments of the present invention, and performing the computer-implemented method.

In a method in accordance with embodiments of the present invention, the step of measuring may comprise weighing the body.

In a method in accordance with embodiments of the present invention, the step of measuring may comprise measuring a length of the body.

In a method in accordance with embodiments of the present invention, the step of measuring may comprise determining a fat component of the body and/or a bone component of the body and/or a muscle component of the body.

In a method in accordance with embodiments of the present invention, the step of measuring may comprise determining a surface area of at least one segment of the body, and/or determining a volume of at least one segment of the body.

In a further aspect, the present invention relates to a device for predicting and/or simulating a plurality of temperatures in a human or animal body when said body is in a predetermined environment (e.g. a controllable or controlled environment). The predetermined environment comprises controllable heating and/or cooling means for bringing a core body temperature of said body to a predetermined target temperature, and/or for maintaining the core body temperature of the body at said predetermined target temperature. The device comprises an input for receiving at least one property of said body obtained by measurements of the body, and a processor.

The processor is adapted for calculating (e.g. configured to calculate) at least one body-specific parameter of a bioheat model based on the at least one property of the body. The processor is adapted for obtaining (e.g. configured to obtain) at least one environment-specific parameter that is indicative of at least one thermal property of said environment and/or of at least one property of a thermal exchange between said body and said environment. The processor is adapted for calculating (e.g. configured to calculate) said temperatures in the body by numerically solving said bioheat model taking said body-specific parameters and said environment-specific parameters into account.

The bioheat model comprises a plurality of equations representative of an equivalent thermal circuit for modeling heat flows in the body and between the body and said environment. The equivalent thermal circuit comprises a blood compartment and a plurality of anatomical segments. The plurality of anatomical segments comprises at least a lower extremity segment, an upper extremity segment, a trunk segment and a head segment. Each of the anatomical segments comprises a plurality of compartments. This plurality of compartments (for each anatomical segment) comprises at least one interior compartment representative of a core and/or bone part of the anatomical segment, at least one intermediate compartment representative of a muscle and/or fat part of the anatomical segment and an exterior compartment representative of a skin part of the anatomical segment.

The at least one interior compartment of the (or at least one of the) at least one trunk segment comprises a plurality of organ compartments representative of respectively at least two internal organs. The at least two organ compartments are connected in parallel in the equivalent thermal circuit.

In a further aspect, the present invention relates to a hyperthermia treatment planning workstation comprising a device in accordance with embodiments of the present invention.

In a further aspect, the present invention relates to a hyperthermia treatment system, comprising a device in accordance with embodiments of the present invention, a controllable environment comprising controllable heating and/or cooling means for bringing a core body temperature of said animal or human body to a predetermined target temperature, and a plurality of temperature sensors for measuring temperatures on or in the body.

A hyperthermia treatment system in accordance with embodiments of the present invention may comprise a hyperthermia treatment planning workstation in accordance with embodiments of the present invention.

In a hyperthermia treatment system in accordance with embodiments of the present invention, the device in accordance with embodiments of the present invention may be adapted for receiving (e.g. configured to receive) sensor data from the plurality of temperature sensors.

In a hyperthermia treatment system in accordance with embodiments of the present invention, the device in accordance with embodiments of the present invention may be adapted for taking (e.g. configured to take) said sensor data into account for calculating said temperatures in the body.

In a hyperthermia treatment system in accordance with embodiments of the present invention, the device in accordance with embodiments of the present invention may be adapted for controlling (e.g. configured to control) said controllable heating and/or cooling means in response to said calculated temperatures in the body.

In a further aspect, the present invention relates to a computer-program product for, when executed on a computing device, performing a method in accordance with embodiments of the present invention.

While the invention is described in the present disclosure with reference to specific embodiments of methods and specific embodiments of devices and of systems, this was done to clarify and not to limit the invention. The skilled person will appreciate that options and features only described with reference to one category of embodiments, e.g. a method, also apply to other embodiments, e.g. embodiments of other methods, devices, and systems.

The independent and dependent claims describe specific and preferred features of the invention. Features of the dependent claims can be combined with features of the independent claims and with features of other dependent claims as deemed appropriate, and not necessarily only as explicitly stated in the claims.

**Brief Description of the Drawings**

FIG 1 illustrates a method in accordance with embodiments of the present invention.
FIG 2 illustrates a further method in accordance with embodiments of the present invention.
FIG 3 illustrates a model for use in embodiments of the present invention.
FIG 4 illustrates a part of an exemplary model for use in embodiments of the present invention.
FIG 5 illustrates a device in accordance with embodiments of the present invention.

The drawings are schematic and not limiting. Elements in the drawings are not necessarily represented on scale. The present invention is not necessarily limited to the specific embodiments of the present invention as shown in the drawings.

**Detailed description of the invention**

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

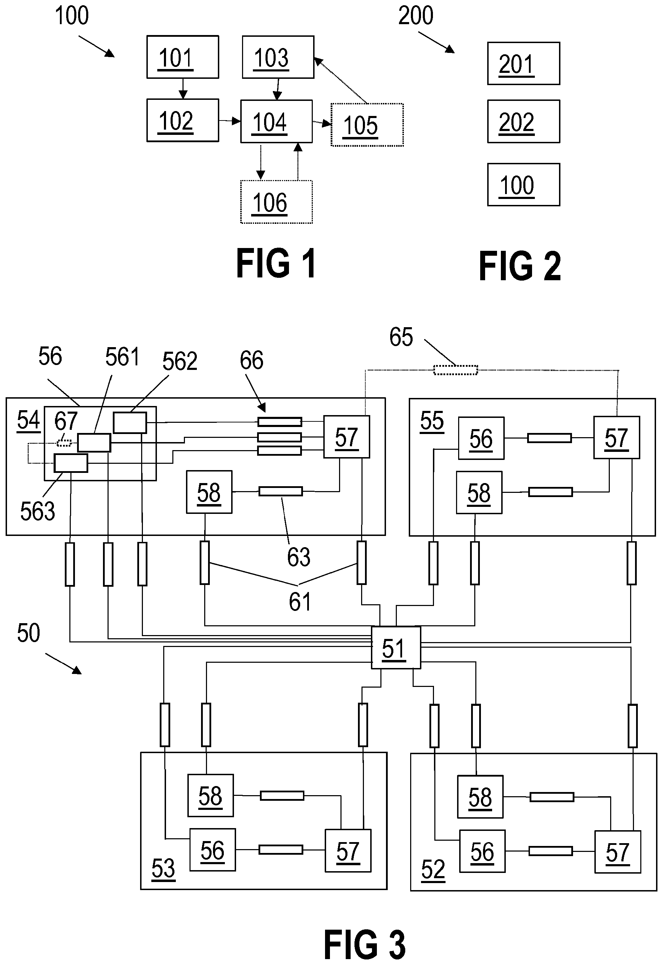

Referring to FIG 1, the present invention relates to a computer-implemented method 100 for predicting and/or simulating a plurality of temperatures in a human or animal body when that body is in a controllable environment, e.g. temperatures of specific body parts, preferably comprising temperatures of specific organs. The controllable environment comprises controllable heating and/or cooling means, heaters and/or coolers, for bringing the core body temperature of the body to a predetermined target temperature. For example, the controllable environment may comprise a hyperthermia treatment system (embodiments not necessarily limited thereto). Such hyperthermia treatment system may comprise an enclosure, e.g. a cabinet, for containing the body, e.g. for fully containing the body when the enclosure is substantially closed around the body. The hyperthermia treatment system may comprise heating and/or cooling means for heating and/or cooling a medium to transfer heat to and/or from the body by convection, e.g. by means of a heated and/or cooled flow of air, of a gas, or of a liquid, e.g. water. The hyperthermia treatment system may comprise heating and/or cooling means for heating and/or cooling the body by radiative heat transfer, e.g. by absorbing heat radiation emitted by the body and/or by emitting radiation that impinges on the body for heating the body, e.g. infrared and/or ultrasonic radiation. The hyperthermia treatment system may comprise heating and/or cooling means for heating and/or cooling the body by heat conduction, e.g. a heated and/or cooled shelf on which the body is positioned, heated and/or other devices for contacting the body (at least locally) and configured to generate or absorb heat, e.g. preferably being actively heated or cooled in response to a control signal.

The hyperthermia treatment system may comprise heating and/or cooling means for heating and/or cooling the body by manipulating the temperature of a body fluid, e.g. of the inhaled and exhaled air and/or of the blood (e.g. by means of an extracorporeal blood circulation device, or an interventional device, for heating and/or cooling the blood flow). Preferably, the hyperthermia treatment system is a full-body hyperthermia treatment system, e.g. configured to raise and maintain the core body temperature at a predetermined value (which is typically elevated with respect to the typical homeostatic temperature of the species).

The method 100 uses a bioheat model of the body. The bioheat model comprises, e.g. can be expressed as, a plurality of equations representative of an equivalent thermal circuit 50, see e.g. FIG 3, for modeling heat flows in the body and between the body and the controllable environment.

The plurality of equations may comprise, or consist of, an equation for each 'node' having (at least a) a specific temperature variable associated therewith. This equation may express a sum of rates of heat transfer (summed over all modelled heat transfer processes involving that node) to (or from) the 'node', which sum may be equated to either zero (assuming a steady-state model) or to, for a transient model, a term proportional to a rate of temperature change, e.g. a product of mass associated with the 'node', a specific heat associated with the 'node' and a partial temporal derivative of the node temperature (or its discrete approximation). For the sake of simplicity and consistency, references to 'heat' hereinbelow are assumed to refer to a rate of heat transfer, e.g. expressed in SI units of Watt, and the symbol Q will be used for such rate of heat transfer (instead of the more conventional symbol Q).

The equivalent thermal circuit may refer to a diagram in which nodes, having a temperature (e.g. $T_b$, $T_{i,j}$) associated therewith, are interconnected by thermal resistances to exchange heat. It will be clear to the skilled person that the set of equations may include heat exchange terms that are not necessarily representable by a thermal analogue of a resistor, capacitor, current source, voltage source or other electrical component. The 'equivalent thermal circuit' thus refers to the similarity of such diagram to an electrical diagram, in which heat flow is analogous to electrical current and temperature is analogous to the electric potential, i.e. voltage. Therefore, as is well-known in the art, the model may comprise an equation for each node that expresses a sum of heat flows by which the node exchanges heat with nodes connected to that node in the diagram (i.e. a sum of rates of heat exchanged). The equation may comprise an additional term(s) indicative of heat generated in that node, and/or may comprise an additional term(s) indicative of heat lost in that node, e.g. by exchange with the environment. The sum may be equated to zero to express a balance of energy in each node. For example, the bioheat model may be solved to obtain a steady-state solution, e.g. by assuming a zero-sum of heat terms for each node. Alternatively, the sum may be equated to a term expressing a change in temperature over time, e.g. may be equated to a term that is proportional to a rate of change of temperature.

Furthermore, each node (or at least some nodes) may have, in addition to a temperature, one or more additional properties attributed thereto, e.g. a mass, a volume $V_{i,j}$, a density $\rho_{i,j}$, a specific heat $c_{i,j}$, a thermal conductivity $k_{i,j}$, a blood perfusion $\omega_{i,j}$, and/or a basal metabolism $Q_{b(i,j)}$ (e.g. expressed in power generated per unit of mass).

While many of the properties assigned to the nodes, such as densities, specific heat, thermal conductivity, basal blood perfusion, basal metabolic heat generation and/or evaporative losses, may be based on values known in the art, or may be determined (theoretically or empirically) for the species (human or animal), in accordance with embodiments of the present invention, at least some of the properties assigned to the nodes are determined from measurements obtained from the specific body, such as dimensions, weight and/or body composition.

As is known in the art, some of the parameters described hereinabove may also be modeled as a variable dependent on (a) further parameter(s), instead of as a fixed parameter as such. For example, the body temperature regulation mechanism may be neglected, in order to simplify the model, or may be modeled explicitly as a mechanism of changing physiological parameters in the human body, such as flood flow, evaporation and metabolism, in response to cold or hot stimuli, e.g. a difference of core and/or skin temperature relative to a reference point. As is known in the art, the reference point, or "set point", of the core temperature and the reference point, or "set point", of the skin temperature may be regulated by a feedback loop as well. However, for applications in which the body is anesthetized, the latter feedback may advantageously be neglected. For an example of a possible implementation of a thermoregulation model, reference is made to Xiang et al, "Comprehensive evaluation on the heating capacities of four typical whole body hyperthermia strategies via compartmental model," in International Journal of Heat and Mass Transfer 51(23-34), pp. 5486-5496, section 3. Embodiments of the present invention may explicitly include the thermoregulation model described in the reference cited hereinabove.

One or more nodes in the equivalent thermal circuit 50 may form a 'compartment', which models a specific part of the body's anatomy. A group of compartments may be grouped together to represent a specific part of the gross body anatomy, which are referred to as anatomical segments. Hereinbelow, the blood compartment is identified by an index $b$, e.g. in the blood temperature $T_b$, while other nodes are identified by an index $i$ signifying the anatomical segment of that node and an index $j$ for enumerating the nodes in that segment, e.g. in the node temperature $T_{i,j}$.

The equivalent thermal circuit 50 comprises a node 51 representative of a blood compartment of the body. The blood can be considered as an important medium for heat transfer between different parts of the body and different tissues. In a good approximation, thermal equilibrium can be assumed between the temperature $T_{i,j}$ associated with each node in the model, e.g. an organ or compartment of a body segment, and the temperature of the blood leaving that node, and the blood can be assumed to enter each node at a same blood temperature $T_b$. This node 51 may have a blood temperature $T_b$ associated therewith (in the plurality of equations of the model). The node 51 may be connected to the other nodes of the model by a plurality of thermal resistances 61. The thermal resistances 61 may have, i.e. typically have, different resistance values attributed thereto, depending on the other node it connects the blood compartment to. Even though, for the sake of clarity, not all thermal resistances are labeled in FIG 3 by the reference 61, it shall be clear that each of the narrow box shapes intersecting a connecting line between the blood compartment node 51 and another node indicates such a thermal resistance 61. The thermal resistance 61 may be representative of a convective heat transfer between the blood perfusing through the body tissue(s) represented by node (i,j) and that body tissue(s). However, in a method in accordance with embodiments of the present invention, some of thermal resistances 61 may be negligible or zero, and therefore not included in the model. For example, for a node representative of a bone compartment of a body segment, blood perfusion may be assumed to be negligible, and therefore heat transfer between the blood and such node may be neglected to simplify the model.

The thermal resistance $R_{b\rightarrow i,j}$ between the blood compartment 51 and another node *(i,j)* may be expressed by $R_{b\rightarrow i,j}^{-1} = \rho_{i,j} V_{i,j} \omega_{i,j} \rho_b c_b$, in which $\rho_{i,j}$, $V_{i,j}$ and $\omega_{i,j}$ represent respectively the density, volume and blood perfusion rate of the node (i,j), and $\rho_b$ and $c_b$ represent the density and specific heat of blood respectively.

For example, the set of equations may comprise an equation for the blood compartment of the form:

$$\sum_{i=1}^{N} \sum_{j=1}^{M_i} R_{b\rightarrow i,j}^{-1} \cdot \left( T_{i,j} - T_b \right) + Q_{in} = \rho_b V_b c_b \frac{\partial T_b}{\partial \tau},$$

where $N$ refers to the number of anatomical segments and $M_i$ refers to the number of nodes in segment $i$. In case the simulation aims for a steady-state prediction, the right-hand side of this equation may alternatively be equal to zero (in such steady-state model, this may clearly apply to all exemplary equations having a similar term discussed further hereinbelow). In case a transient simulation is intended, the term $\frac{\partial T_b}{\partial \tau}$ refers to a partial derivative (or a discrete numeric approximation thereof) of the blood temperature with respect to time $\tau$. The term $Q_{in}$ is optional, and represents an exogenous heating factor, e.g. by controlled heating (or cooling) of the blood via extracorporeal blood circulation or interventional means.

However, embodiments of the present invention are not necessarily limited to this example. As is known in the art, the blood flow model may be modeled in greater detail, e.g. by coupling the conduction and perfusion-based terms of the example discussed in the present disclosure to a model of arterial fluid dynamics. For example, the blood compartment may be divided in a plurality of compartments corresponding to different arterial sections. Reference is made to Coccarelli et al., "An advanced computational bioheat transfer model for a human body with an embedded systemic circulation," in Biomechanics and Modeling in Mechanobiology 15(5), pp. 1173-1190. Embodiments of the present invention may comprise the features of embedded systemic circulation model discussed in the aforementioned reference.

The equivalent thermal circuit 50 comprises a plurality of anatomical segments, the plurality of anatomical segments comprising at least one lower extremity segment 52, at least one upper extremity segment 53, at least one trunk segment 54 and at least one head segment 55.

It shall be clear to the skilled person that the model may comprise two lower extremity segments, representative of a left leg and a right leg respectively, or may comprise a single lower extremity segment, representative of either leg (e.g. of both legs collectively or of an 'average' leg representative of either leg), in which a factor 2 may be applied in the set of equations, where appropriate, to take into account the presence of two lower extremities in the human or animal body. Obviously, the same may apply to the upper extremities.

It shall also be clear that parts of the extremities may be modeled as separate segments, hence the reference to 'at least one' lower extremity segment 52 and at least one upper extremity segment 53. For example, a separate segment may be included for the hand and the arm, and/or a separate segment may be included for the foot and the leg. Furthermore, parts of the arm and/or the leg may be further subdivided into anatomical segments, e.g. a lower arm and an upper arm or shoulder segment, and/or a lower leg and an upper leg or thigh segment.

Likewise, it shall be clear to the skilled person that the at least one head segment 55 may be further divided into a plurality of segments, e.g. a head segment and a neck segment.

Likewise, it shall be clear to the skilled person that the at least one trunk 54 may be further divided into a plurality of segments, e.g. a thorax segment and an abdomen segment.

For example, the plurality of segments may comprise or consists of: a head segment, a neck segment, a thorax segment, an abdomen segment, a left leg segment, a right leg segment, a left foot segment, a right foot segment, a left arm segment, a right arm segment, a left hand segment and a right hand segment. It shall be clear that this is merely exemplary, and the number of different segments that are explicitly modeled may vary across embodiments, e.g. depending on a need for accuracy with respect to a trade-off for speed, efficiency and simplicity, as well as possible anatomical variations over animal (including human) species.

Each of the anatomical segments 52, 53, 54, 55 comprises a plurality of compartments comprising at least one interior compartment 56 representative of a core and/or bone part of the anatomical segment, at least one intermediate compartment 57 representative of a muscle and/or fat part of the anatomical segment and an exterior compartment 58 representative of a skin part of the anatomical segment.

It shall be clear to the skilled person that the thermal equivalent circuit 50 of the model may include further nodes, e.g. a node to model the environment or elements of the environment, or nodes which are logically grouped with the segments to model effects that are not inherently associated with the body, e.g. the effect of an external layer (e.g. situated outward with respect to the exterior compartment 58) for taking the effect of a thermal insulator, such as clothing, into account.

Each of the nodes of the diagram forming the compartments of a single anatomical segment may have a corresponding temperature associated therewith, e.g. $T_{i,j}$, in which the index $i$ refers to a segment, and the index $j$ refers to a node in that segment (or compartment of the segment, insofar each compartment consists of a single node for that segment).

The interior, intermediate and exterior compartments for each anatomical segment may be serially connected, in a sense from outermost to innermost, by a plurality of thermal resistances, e.g. an interior compartment 56 may be connected over a thermal resistance 62 to an intermediate compartment 57, and an intermediate compartment 57 may be connected over a thermal resistance 63 to an exterior compartment 58. When a plurality of intermediate compartments 571,572 are included in the model for a segment, these may be connected in series as well. However, when a plurality of interior compartments 561,562,563 are included in the model for a segment, these may be preferably connected in parallel, e.g. such that each interior compartment is connected to the intermediate compartment (or to the innermost intermediate compartment) over a separate thermal resistance. In accordance with embodiments of the present invention, the (or some of the) interior compartments of a segment may also be connected to each other via further thermal resistances.

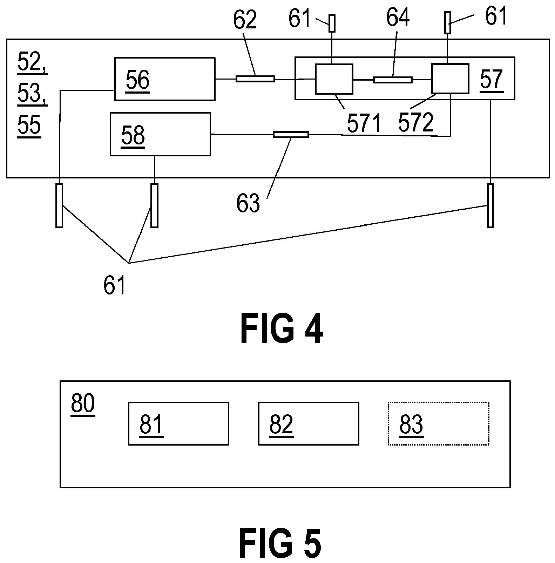

Referring to FIG 4, preferably, each anatomical segment may comprise an interior compartment 56, two intermediate compartments 571,572 for respectively modeling a muscle part of the anatomical segment and a fat part of the anatomical segment, and an exterior compartment 58. The two intermediate compartments 571,572 may be connected to each other via a thermal resistance 64. Thus, the four compartments 56, 571, 572 and 58 may be connected in series by respectively the thermal resistances 62, 64, 63. It shall be clear that this is exemplary, and the number of different layers (intermediate compartments between the outermost skin layer and the internal core) that are modeled may vary across embodiments.

The thermal resistances 62,64,63 between the compartments of a segment may be representative of heat conduction between the corresponding pair of compartments.

As will be clear to the skilled person, in embodiments in accordance with the present invention, optionally, (an) additional thermal resistance(s) 65 may be included to model conductive heat flows between corresponding compartments of segments that are connected in the body, e.g. (without limitation) between the interior compartment of the head and the interior compartment of the neck, or between an intermediate compartment 56 (e.g. a muscle compartment) of the (or a) head segment 55 (e.g. the neck segment) and a corresponding intermediate compartment 56 the (or an) trunk segment 54 (e.g. the thorax segment).

For example, the set of equations may comprise an equation for each intermediate compartment 57 that expresses a total heat balance in the compartment, either assuming steady-state (in which a partial temporal derivative of temperature would be assumed zero) or in a transient model (in which the net heat transfer rate would be equated to a proportional change in temperature). This equation may comprise a term representative of heat exchange with the blood compartment by convection, e.g. similar (e.g. oppositely signed) to the term in the equation for the blood compartment discussed hereinabove, terms representative of the heat exchange with its adjacent compartments by conduction and a metabolic heat generation term. For example, this equation may be of the form:

$$R_{b \to i,j}^{-1} \cdot \left( T_b - T_{i,j} \right) + Q_{cond}(i, j-1) - Q_{cond}(i,j) + Q_{met}(i,j) = \rho_{i,j} V_{i,j} c_{i,j} \frac{\partial T_{i,j}}{\partial \tau},$$

where $Q_{cond}(i,j)$ and $Q_{cond}(i,j-1)$ represents the heat flow by conduction (represented by the corresponding thermal resistance 62,64,63) to the next (in the series) compartment and from the previous (in the series) compartment respectively, and $Q_{met}(i,j)$ represents metabolic heat generated in the compartment $(i,j)$.

The terms representative of heat flow by conduction between a pair of adjacent compartments, i.e. representative of the thermal resistances 62,63,64,65,66,67 in the thermal equivalent circuit, can be expressed as $Q_{cond}(i,j) = K_e \cdot (T_{i,j} - T_{i,j+1}) = (T_{i,j-1} - T_{i,j})/R_{i,j}$, where $K_e$ is the effective heat conductance between the adjacent tissue layers. This effective heat conductance can be expressed by the Ohm's law analogue: $1/K_e = \dfrac{1}{K_e(i,j)} + \dfrac{1}{K_e(i,j+1)}$. A geometrical model can be used to estimate the effective heat conductance. For example, for body segments which may be assumed to be (approximately) cylindrical: $\dfrac{1}{K_e(i,j)} = \dfrac{2\pi K(i,j)L(i,j)}{\ln\left(\frac{r_{b,j}}{r_{mid,j}}\right)}$ and $\dfrac{1}{K_e(i,j+1)} = \dfrac{2\pi K(i,j+1)L(i,j+1)}{\ln\left(\frac{r_{mid,j+1}}{r_{b,j}}\right)}$, and for body segments which may be assumed to be (approximately) spherical: $\dfrac{1}{K_e(i,j)} = \dfrac{4\pi K(i,j)}{1/r_{mid,j}-1/r_{b,j}}$ and $\dfrac{1}{K_e(i,j+1)} = \dfrac{4\pi K(i,j)}{1/r_{b,j}-1/r_{mid,j+1}}$, where $K(i,j)$ refers to the thermal conductivity of the element $(i,j)$, $L(i,j)$ refers to the length of the element $(i,j)$, and $r_{b,j}$ and $r_{mid,j}$ refer respectively to the radius at the boundary and mid-volume of the element.

The term representative of metabolic heat rate generated in the compartment may be expressed as a product of a basal metabolism term $Q_{b(i,j)}$ and the mass of the compartment (which may be estimated from a density and volume of the compartment).

The set of equations may comprise an equation for each exterior compartment 58. The equations in the model for the exterior compartments 58 may include one or more terms connecting the exterior compartment to the controllable environment. These equations for the exterior compartments may have a form that is similar to the equation for the intermediate compartment(s), for example:

$$R_{b\rightarrow i,j}^{-1} \cdot \left(T_b - T_{i,j}\right) + Q_{cond}(i,j-1) + Q_{met}(i,j) - Q_{evap}(i) - Q_{rad} - Q_{conv} - Q_{cond,E,i} =$$

$$\rho_{i,j} V_{i,j} c_{i,j} \frac{\partial T_{i,j}}{\partial \tau},$$

except for, obviously, not including a heat flow $Q_{cond}(i,j)$ by conduction to a further compartment of the body segment that would be located further outward, and, instead, including one or more further terms representative of heat exchange with the controllable environment via the exterior surface of the body, e.g. via the skin. These further terms may comprise (e.g. one of, all of, or any combination of) a term for evaporative heat loss $Q_{evap}(i)$, a term for radiative heat exchange $Q_{rad}$ with the environment, a term for convective heat exchange $Q_{conv}$ with the environment, and a term for conductive heat exchange with the environment $Q_{cond,E,i}$.

For example, the convective heat exchange term may depend on an exposed area A of the exterior surface of the body segment, on a convection coefficient $h_{air}$ and/or on the air temperature $T_{air}$ of the environment, e.g. $Q_{conv} = Ah_{air}(T_{i,j} - T_{air})$. The convective coefficient ($[W/m^2K]$) expresses how effective the convective cooling or heating of the body is, and may depend on the specific environment. For example, for forced convective cooling and/or heating, e.g. by a air flow driven by a fan, the convective coefficient may depend on the speed and characteristics of the fan. This parameter may be determined experimentally (or by fluid mechanical simulation), e.g. as function of one or more convection control parameters of the controllable environment, e.g. as function of fan speed.

While the convective coefficient may be considered a constant, or may be considered as a parameter dependent on environment-specific parameters, e.g. a fan speed and fan air temperature, embodiments of the present invention are not necessarily limited thereto. For example, the convective coefficient may differ for each body segment, e.g. as determined by a body posture, clothing or other factors. A relationship may be established, theoretically or empirically, between the convective coefficient for each body segment and external controllable parameters, i.e. one or more of the environment-specific parameters.

The radiative heat exchange term may depend on the exposed area A of the exterior surface of the body segment, emittance $\varepsilon$ of the body surface and the surrounding temperature $T_{sur}$ in the environment, e.g. $Q_{rad} = A\varepsilon\sigma\left(T_{i,j}^4 - T_{sur}^4\right)$, where $\sigma$ is the Stefan-Boltzmann constant.

The evaporative heat exchange term may be assumed constant for each body segment (but not necessarily equal for two different body segments), for example based on values reported in literature. However, the evaporative heat exchange term may also be experimentally determined as function of environmental conditions, e.g. of ambient temperature and/or air humidity. Furthermore, in an application in which the body is anesthetized, e.g. for a hyperthermia treatment, the evaporative heat exchange term may be neglected, e.g. due to absence of sweating under anesthesia.

The conductive heat exchange term $Q_{cond,E,i}$ may express a conductive heat transfer by contact of the exterior surface of the body segment with a heated or cooled surface, e.g. a (controllably) cooled or heated shelf on which the (or part of) the body resides, or a heating/cooling device contacting a part of the body.

Likewise, the set of equations may comprise an equation for each interior compartment 56, which may have a similar expression,

$$R_{b\to i,j}^{-1}\cdot\left(T_b - T_{i,j}\right) - Q_{cond}(i,j) + Q_{met}(i,j) = \rho_{i,j}V_{i,j}c_{i,j}\frac{\partial T_{i,j}}{\partial\tau},$$

except for, obviously, not receiving a heat flow $Q_{cond}(i,j-1)$ by conduction from a further compartment that would be located further inward. Furthermore, the equation for the interior compartment 56 may also include an additional term for respiratory heat loss, $Q_{resp}$.

Furthermore, the interior compartment of segments such as the neck and the extremities (or parts thereof, e.g. a leg, arm, hand or foot) may be representative of bone tissue, which may be assumed to have a negligible blood perfusion and metabolic heat generation associated therewith. Therefore, the equation for the interior compartment of such segments having a central bone may be simplified as:

$$-Q_{cond}(i,j) = \rho_{i,j}V_{i,j}c_{i,j}\frac{\partial T_{i,j}}{\partial\tau}.$$

The interior compartment of a trunk segment, e.g. of a chest (i.e. thorax) segment, may include a further term $Q_{resp}$ representative of respiratory heat transfer, e.g.

$$R_{b\to i,j}^{-1}\cdot\left(T_b - T_{i,j}\right) - Q_{cond}(i,j) + Q_{met}(i,j) - Q_{resp} = \rho_{i,j}V_{i,j}c_{i,j}\frac{\partial T_{i,j}}{\partial\tau},$$

In which the respiratory term may be based on a respiratory heat transfer model known in the art. For example, $Q_{resp}$ = 0.0023 $Q_{met}(44 - p_a)$, where $p_a$ refers to the partial pressure (in mmHg) of water vapor in the inspired air and $Q_{met}$ is a metabolic heat production rate parameter.

Furthermore, the at least one interior compartment 56 of a trunk segment 54, e.g. of an abdomen segment, comprises (or consists of) at least two organ compartments 561,562 representative of respectively at least two internal organs of the body in the trunk region, such as the heart, the liver, the kidneys (or a kidney, or each kidney), the stomach, the pancreas, the lungs, the intestines (possibly divided over multiple compartments), the gallbladder, the bladder, the ovaries (or each ovary), and/or the uterus. Preferably, the at least two organ compartments comprise at least a compartment representative of the liver.

Furthermore, the at least one interior compartment 56 of the trunk segment 54 that comprises the at least two organ compartments 561,562 may, preferably, also comprise a remainder compartment 563 representative of the remainder of the interior part of the trunk segment, e.g. the remainder of the interior (e.g. interior in the sense of being situated inward with respect to the innermost intermediate compartment of that segment) of that trunk segment, e.g. of the abdomen segment, which remains after all the other organ compartments representative of specific organs are accounted for.

The plurality of organ/remainder compartments 561,562,563 are connected in parallel in the equivalent thermal circuit, i.e. each connected separately to the innermost intermediate compartment, e.g. a muscle compartment, of that trunk segment 54, e.g. of the abdomen segment, via a corresponding thermal resistance 66 representative of heat conduction between the organ (or remainder) and the intermediate compartment. As is the case for all compartments of the model, with the possible exception of compartments representative of bone, each of the organ compartments is connected to the blood compartment 51 via a thermal resistance 61 representative of a convective heat transfer between the blood perfusing through the organ (or remainder part of the trunk segment, for the organ compartment representative of the remainder of the interior of the trunk segment).

Thus, the set of equations may comprise for each interior compartment that is an organ/remainder compartment of such trunk region an equation, such as:

$$R_{b\to i,j}^{-1}\cdot\left(T_b - T_{i,j}\right) - Q_{cond}(i,j) + \cdots + Q_{met}(i,j) = \rho_{i,j}V_{i,j}c_{i,j}\frac{\partial T_{i,j}}{\partial\tau}$$

**EP 3 751 577 A1**

which may optionally also include additional terms (illustrated by the ellipsis sign in the equation hereinabove) representative of rates of heat flow between the, or some of the, internal organs (and/or remainder). Therefore, the equivalent circuit may, optionally, comprise one or more thermal resistances 67 representative of thermal conduction between organ compartments corresponding to adjacent organs.

However, since the plurality of organ/remainder compartments are connected in parallel to the innermost intermediate compartment, e.g. the muscle layer of the abdomen, the equation for this compartment will reflect this accordingly. In the exemplary equation provided hereinabove for a generic intermediate compartment, the change may take the form of:

$$R_{b \to i,j}^{-1} \cdot \left(T_b - T_{i,j}\right) + \sum_{k=1}^{n_{organs}} Q_{cond}(i, j - k) - Q_{cond}(i, j) + Q_{met}(i, j) = \rho_{i,j} V_{i,j} c_{i,j} \frac{\partial T_{i,j}}{\partial \tau},$$

In which k iterates over the number of organ compartments $n_{organs}$.

Embodiments of the present invention are not necessarily limited to the latter example. It may be advantageous to include a more detailed model of the (e.g. each) organ. For example, Pennes' bioheat transfer equation, as known in the art, may be used.

It is an advantage of embodiments of the present invention that, by modeling the core of a trunk region, e.g. of an abdomen region, by parallelly connected organs (and possibly including a separate component for the remainder of te core), variations in temperature for the organs can be simulated accurately. By modelling these organs separately, each organ can be assigned its own specific heat, thermal conductivity, basal blood perfusion rate, and/or basal metabolism as parameter(s), to improve accuracy of the simulation.

The thermal resistances 66 can be determined by taking an estimate of the organ mass (or of the remainder of the segment core) and a contact fraction into account, in which the contact fraction represents the portion of the interior surface of the innermost intermediate compartment (e.g. muscle layer) that is assumed to be in direct thermal contact with the organ (or resp. the remainder component of the core). Obviously, the organ mass can be equivalently expressed as a fraction of the total core of the body segment, e.g. as a volume or mass fraction of the abdominal cavity filled by the organ (resp. the remainder component).

These organ masses and contact fractions can be obtained from knowledge in the art, may be experimentally determined for a population (e.g. species), or may be determined as a subject-specific parameter, e.g. by segmentation of medical images of the specific body.

Furthermore, it is an advantage of using a remainder compartment 563, a consistent and easily constructed model can be achieved. For example, referring to the exemplary method of determining thermal resistances based on a simple geometrical model of the body segment as multiple concentric compartments of a simple geometric shape, the thermal resistance $R_{core}$ of the core of this trunk section can be determined for the case where no internal organ differentiation is applied (as would be the approach in accordance with this example for other body segments). Then, the thermal resistance of the remainder compartment can be easily determined by $R_{remainder} = \left(\frac{1}{R_{core}} - \sum_{k=1}^{n_{organs}} \frac{1}{(R_k)}\right)^{-1}$, and likewise the basal metabolism can be estimated by $Q_{b,remainder} = Q_{b,core} - \sum_{k=1}^{n_{organs}} Q_{b,k}$.

It shall be clear to the skilled person that the at least one trunk segment may comprise an abdomen segment and a thorax segment. The approach described hereinabove can be followed for each of these trunk segments separately, e.g. to model the core of the thorax segment by separate lungs and heart organ compartments and to model the core of the abdomen segment by separate liver, stomach, kidney, ... organ compartments. However, surprisingly, the inventors have found that no internal organ differentiation of the thorax segment in combination with internal organ differentiation in the abdomen segment provide superior results in terms of modelling accuracy and ease of use.

The method 100 comprises receiving 101, as input, at least one property of the body obtained by measurements of the (specific) body. The at least one property of the body may comprise anthropometric data. The at least one property may, particularly, comprise one or more properties indicative of weight (i.e. mass), volume, dimensions and/or composition of the body. For example, the at least one property may comprise a mass of the body (or, equivalently, its weight). The at least one property may comprise a mass, a volume, a fraction, and/or a percentage (e.g. by weight or by volume) of body fat and/or muscle and/or bone in the body. The at least one property may comprise one or more lengths, diameters, surface areas, volumes, and/or other anthropometric measurements of a (or each) lower and/or upper extremity of the body, and/or of the neck, and/or of the head, and/or of the trunk.

Referring to FIG 2, embodiments of the present invention may also relate to a (further) method 200 comprising a step of measuring 201 the at least one property of the body, providing 202 the at least one property of the body as input to the computer-implemented method 100 and performing the computer-implemented method 100.

Measuring the at least one property may comprise weighing the body.

Measuring the at least one property may comprise measuring a length of the body.

Measuring the at least one property may comprise determining a fat mass or fat percentage of the body, e.g. by skinfold measurements (e.g. using a caliper), by estimation based on tape measurements, by bioelectrical impedance measurements, by hydrodensitometry, by air displacement plethysmography, by medical imaging (e.g. CT, MRI, DEXA, or echography), or any other method known in the art for determining a fat component of the body.

Optionally, (an)other body tissue type component(s), such as a bone component and a muscle component, can be determined as well, e.g. using suitable techniques known in the art (e.g. by medical imaging). Alternatively, volumes of such other components can be estimated by reference data, e.g. based on body composition, for example, by using the fat percentage and/or the weight and/or the length of the body (or a quantity derived therefrom) for look-up in such reference data.

Measuring the at least one property may comprise determining a surface area and a volume of each segment of the body (i.e. each body part of the body that corresponds to an anatomical segment of the model).

For example, the surface area and volume of each segment of the body may be determined by medical imaging, e.g. by CT, MRI or DEXA, or 3D body (surface) scanning, e.g. using a laser scanner, optical camera or infrared imaging. Alternatively, the volume of each segment of the body may be determined by submersion or air displacement measurement. The surface area of each segment of the body may be determined by wrapping paper. Alternatively, the volume and surface area of each segment of the body may be estimated from one or more circumference measurements and/or one or more linear projection measurements (e.g. width, depth, and/or height of a bounding box volume), and/or length measurements. For example, a geometrical model of the segment may be assumed, such as a cylindrical shape for the extremities (or parts thereof), the trunk (or parts thereof), and/or the neck, and/or a spherical shape for the head. Embodiments of the present invention are not necessarily limited to cylindrical and/or spherical shapes. For example, shapes such as elliptic cylinders, truncated cones, ellipsoids, boxes and the like can be contemplated.

The method 100 further comprises calculating 102 at least one body-specific parameter of the bioheat model based on the at least one property of the body.

Calculating 102 the at least one body-specific parameter of the bioheat model may comprise determining thermal resistance values (or, equivalently, effective heat conductance values) representative of thermal conduction between compartments of the model, e.g. represented by the thermal resistances 62,63,64,65,66 in the thermal equivalent circuit. These thermal resistance values may be determined based on the measured or estimated surface area and volume of each segment. For example, the effective heat conductance for each compartment (or at least some of the compartments) may be estimated using a geometric model (e.g. see hereinabove) from an equivalent length and/or an equivalent radius (or radii) of the compartment. The equivalent radius and equivalent length of the outer boundary of each body segment can be easily determined from the measured surface area and volume of the segment. For example, assuming a cylindrical model: $A = 2\pi rL$ and $V = \pi r^2 L$, where A and V refer respectively to the measured or estimated area and volume and r and L refer respectively to the equivalent outer radius of the exterior compartment and the equivalent length of the segment. The outer boundary of each intermediate compartment can be likewise determined, by subtracting equivalent thicknesses of the corresponding layer. For example, the outer equivalent radius $r_{i+1}$ of each inwardly adjacent compartment with respect to a compartment with known outer boundary $r_i$ (e.g. starting from the outer radius of the exterior compartment as determined by the equation hereinabove) can be estimated by $r_{i+1} = \sqrt{r_i^2 - \frac{V_i}{\pi L}}$, where the volume $V_i$ of the compartment discounted for can be determined as a fraction from the total volume, e.g. using estimates of skin thickness, fat percentage and muscle percentage.

The method 100 also comprises obtaining 103 at least one environment-specific parameter that is indicative of at least one thermal property of the controllable environment and/or of at least one property of a thermal exchange between the body and the controllable environment.

The at least one environment-specific parameter may comprise a value representative of a convection coefficient $h_{air}$ of an air flow to which the body is exposed in the environment. This value may be the convection coefficient indicating the efficiency of convective cooling (or heating) of the body, or may determine the convection coefficient by a known functional relationship or a correspondence look-up table. For example, the value determining the convection coefficient may be representative of a fan speed or fan setting. However, the convection coefficient does not necessarily relate to a forced air flow (e.g. where an average air flow velocity differs substantially from zero), e.g. may also relate to a convection coefficient indicative of air convection in a static air environment (e.g. where an average air velocity is substantially zero).

The at least one environment-specific parameter may comprise an air temperature or a value representative of an air temperature $T_{air}$ of the environment. This air temperature may represent an ambient air temperature of the environment or may represent an air temperature of the air in a forced air flow, e.g. in which heated or cooled air is blown by a fan onto the body.

For example, the method 100 may comprise calculating a value representative of a rate of convective heat exchange for each exterior compartment 58 based on an exposed area A of the exterior surface of the body segment, on the value representative of the convection coefficient $h_{air}$ and on the value representative of the air temperature $T_{air}$, e.g. $Q_{conv} = Ah_{air}(T_{i,j} - T_{air})$.

The at least one environment-specific parameter may comprise a temperature $T_{sur}$ of the surroundings of the body in the environment, e.g. as can be used in calculations of radiative heat transport. For example, the method 100 may comprise calculating a value representative of a rate of radiative heat exchange for each exterior compartment 58 based on an area $A$ of the exterior surface of the body segment, an emittance $\varepsilon$ of the exterior surface of the body segment and the temperature $T_{sur}$ of the surroundings in the environment, e.g. $Q_{rad} = A\varepsilon\sigma\left(T_{i,j}^4 - T_{sur}^4\right)$, where $\sigma$ is the Stefan-Boltzmann constant.

The at least one environment-specific parameter may comprise a temperature $T_{contact}$ of an actively heated and/or cooled surface contacting at least part of the body in the environment. The method 100 may comprise calculating a value $Q_{cond,E,i}$ representative of a rate of conductive heat exchange for an exterior compartment 58 based on a contact area $A_{contact}$ over which the surface of the corresponding body segment contacts the heated and/or cooled surface and the temperature $T_{contact}$.

The at least one environment-specific parameter may comprise a respiration temperature $T_{resp}$, e.g. a temperature of inspired air. The at least one environment-specific parameter may comprise a relative air humidity $\varphi$, e.g. a humidity of inspired air. The method 100 may comprise calculating a value $Q_{resp}$ representative of a rate of heat exchange by respiration, e.g. $Q_{resp} = 0.0023\ Q_{met}(44 - p_a)$, where $p_a$ refers to the partial pressure of water vapor in the inspired air and $Q_{met}$ is a metabolic heat production rate parameter. The partial pressure of water vapor can be determined based on the relative humidity, $p_a = \varphi.p_s$, in which the saturation pressure $p_s$ can be determined based on the temperature $T_{resp}$. For example, without limitation, the saturation pressure can be estimated by Tetens equation: $p_s = 0.61078e^{\frac{17.27 T_{resp}}{T+237.3}}$.

However, embodiments of the present invention are not necessarily limited to this example. Respiratory heat losses may also be modeled as a sum of convective and evaporative heat transfer, as is known in the art, which may be useful when considering both air temperature and relative humidity of the inhaled air as variable environment-specific parameters.

The at least one environment-specific parameter may comprise a thermal property, such as a power or temperature set-point, of a heater and/or cooler for heating and/or cooling the blood via extracorporeal blood circulation or interventional means.

The method 100 further comprises calculating 104 the temperatures in the body by numerically solving the bioheat model, in which the body-specific parameter(s) and the environment-specific parameter(s) are taken into account. For example, calculating 104 the temperatures may comprise solving (e.g. jointly solving) the plurality of equations representative of the equivalent thermal circuit 50 in accordance with a steady-state requirement. For example, such steady-state requirement may be a requirement of these temperatures being constant, i.e. such that a temporal derivative of the temperatures can be equated to zero (and thus, removed as variables to solve). Therefore, in such steady-state scenario, each of the equations may express a sum of rates of heat transfer that is equated to zero, and the problem can be solved by minimizing an error, in which this error expresses a deviation from this zero balance state, e.g. a sum (over the number of equations) of error terms (e.g. square of the sum of heat transfer rates).

In a method 100 in accordance with embodiments of the present invention, the step of obtaining 103 the at least one environment-specific parameter may comprise obtaining a plurality of different values of the environment-specific parameter, and performing the step of calculating 104 the temperatures for each value of the environment-specific parameter to determine changes in the temperatures as dependent on the parameter. It shall be clear that this can be extended to evaluating the influence of multiple parameters.

Obtaining 103 the at least one environment-specific parameter may also comprise adjusting 105 a previously evaluated value of the at least one environment-specific parameter to optimize one or more of the temperatures. For example, an optimization strategy as known in the art (e.g. based on gradients or Hessian matrices, direct search strategies, neural networks or other machine learning strategies, etc.) may be applied to determine the at least one environment-specific parameter (or combination of environment-specific parameters) that provides a solution of the temperatures that is close to a target solution of the temperatures, e.g. the predetermined target temperature. For example, for a hyperthermia simulation, the temperature of the blood may be optimized to equal (or approximate) a predetermined value, e.g. 41.5°C, or the temperature of a specific tumor node may be optimized to equal (or approximate) a predetermined value.

Furthermore, the objective of such optimization strategy may also comprise hard and/or soft constraints on at least one of the temperatures. For example, an upper tolerable temperature limit may be set for a temperature(s) of the organ compartment(s). Methods are known in the art to accommodate such constraints in the numeric optimization, e.g. by means of Lagrange multipliers.

Thus, it is an advantage of embodiments of the present invention that a (combination of) environment-specific parameter(s) of a controllable environment can be determined to achieve a predetermined target temperature(s) in the body, e.g. while ensuring that constraints are met to avoid or reduce the risk of organ damage.

Calculating 104 the temperatures may comprise solving (e.g. jointly solving) the plurality of equations representative of the equivalent thermal circuit 50 in a transient manner, e.g. solving for a plurality of time points. For example, starting from a steady-state solution, a parameter, such as a environment-specific parameter may be perturbed (e.g. slightly changed) to determine the impact on the temporal derivatives of the temperatures. Then, a next time step can be simulated by updating the temperatures in the model in accordance with the temporal derivatives. It shall be clear that many other approaches, of various complexities, are known in the art for simulating such transient models, and are considered suitable for the purposes of the present invention.

For example, while the body could be brought to a hyperthermia state by using one or more sources of heat, set in accordance with parameters obtained from a steady-state simulation, a more efficient approach may be determined by solving a transient model. This approach, therefore, advantageously enables a more rapid and/or controlled warming-up of the body. For example, in an early phase of the treatment, the body may be heated relatively rapidly, while at a later phase of the treatment, external heating may be (e.g. gradually) switched to external cooling to evacuate the energy generated by the basal metabolism once the target temperature has been reached.

The method 100 may also comprise receiving 106, as input, sensor data from one or more temperature sensors on or in the body, and taking this sensor data into account when solving the plurality of equations representative of the equivalent thermal circuit 50. For example, temperatures of one or more nodes of the model may be set to a fixed value obtained from the sensor data, or a deviation of a temperature of a node with respect to its measured counterpart derived from the sensor data may be included when solving the equations as a minimization criterion. For example, in a transient model simulation, the sensor data may be used as feedback in the model to increase the accuracy of parameter predictions to achieve a target objective.

Where the present invention is described hereinabove with reference to specific embodiments of a method, this is solely for the sake of clarification and not to be construed as limiting the invention. The skilled person will understand that options and features only described with reference to such methods also apply to a device or system in accordance with embodiments of the present invention, and vice versa.

In a second aspect, the present invention relates to a device for predicting and/or simulating a plurality of temperatures in a human or animal body when the body is in a controllable environment that comprises controllable heating and/or cooling means for bringing a core body temperature of said body to a predetermined target temperature.

Referring to FIG 5, an exemplary device 80 in accordance with embodiments of the present invention is shown. The device 80 comprises an input 81 for receiving at least one property of said body obtained by measurements of the body, and a processor 82.

The processor 82 is adapted for calculating at least one body-specific parameter of a bioheat model based on the at least one property of the body, obtaining at least one environment-specific parameter that is indicative of at least one thermal property of said controllable environment and/or of at least one property of a thermal exchange between said body and said controllable environment, and calculating said temperatures in the body by numerically solving said bioheat model taking said body-specific parameters and said environment-specific parameters into account.

The environment-specific parameter may be received as input, e.g. via the input 81, or may be postulated by the device, e.g. as a trial value in implementing an optimization of the at least one environment-specific parameter to achieve one or more objectives, e.g. the predetermined target temperature and/or a constraint(s) of internal organ temperature(s).

The device may also comprise an output 83 for outputting the calculated temperatures, and/or for outputting an optimized value (or values) of the at least one environment-specific parameter, and/or for outputting a control signal for controlling the controllable environment.

The bioheat model comprises a plurality of equations representative of an equivalent thermal circuit 50 for modeling heat flows in the body and between the body and the controllable environment, in which the equivalent thermal circuit comprises a blood compartment 51 and a plurality of anatomical segments. The plurality of anatomical segments comprise at least a lower extremity segment 52, an upper extremity segment 53, a trunk segment 54 and a head segment 55. Each of the anatomical segments 52,53,54,55 comprises a plurality of compartments, the plurality of compartments comprising at least one interior compartment 56; 561,562,563 representative of a core and/or bone part of the anatomical segment, at least one intermediate compartment 57; 571,572 representative of a muscle and/or fat part of the anatomical segment and an exterior compartment 58 representative of a skin part of the anatomical segment.

The at least one interior compartment of the at least one trunk segment 54 comprises a plurality of organ compartments 561,562 representative of respectively at least two internal organs, and at least two organ compartments 561,562 are connected in parallel in said equivalent thermal circuit.

For further details regarding essential or optional features of the bioheat model and/or calculations in relation thereto, which may be performed by the processor 82, reference is made to the discussion hereinabove on the topic of embodiments of the methods 100 and 200.

In a third aspect, the present invention relates to a hyperthermia treatment planning workstation that comprises a device in accordance with embodiments of the second aspect of the present invention.

In a fourth aspect, the present invention relates to a hyperthermia treatment system comprising a device in accordance with embodiments of the second aspect of the present invention, a controllable environment comprising controllable heating and/or cooling means for bringing a core body temperature of the animal or human body to a predetermined target temperature, and a plurality of temperature sensors for measuring temperatures on or in the body. The device is adapted for receiving sensor data from the plurality of temperature sensors, taking the sensor data into account for calculating the temperatures in the body, and for controlling the controllable heating and/or cooling means is response to the calculated temperatures in the body.

The controllable environment comprises controllable heating and/or cooling means, heaters and/or coolers, for bringing the core body temperature of the body to a predetermined target temperature. The hyperthermia treatment system may comprise an enclosure, e.g. a cabinet, for containing the body, e.g. for fully containing the body when the enclosure is substantially closed around the body. The hyperthermia treatment system may comprise heating and/or cooling means for heating and/or cooling a medium to transfer heat to and/or from the body by convection, e.g. by means of a heated and/or cooled flow of air, of a gas, or of a liquid, e.g. water. For example, the system may comprise a fan for blowing heated (or cooled) air into the enclosure.

The hyperthermia treatment system may comprise heating and/or cooling means for heating and/or cooling the body by radiative heat transfer, e.g. by absorbing heat radiation emitted by the body and/or by emitting radiation that impinges on the body for heating the body, e.g. infrared and/or ultrasonic radiation.

The hyperthermia treatment system may comprise heating and/or cooling means for heating and/or cooling the body by heat conduction, e.g. a heated and/or cooled shelf on which the body is positioned, heated and/or other devices for contacting the body (at least locally) and configured to generate or absorb heat, e.g. preferably being actively heated or cooled in response to a control signal. For example, such heating and/or cooling means may comprise ohmic heater elements and/or Peltier elements.

The hyperthermia treatment system may comprise heating and/or cooling means for heating and/or cooling the body by manipulating the temperature of a body fluid, e.g. of the inhaled and exhaled air and/or of the blood (e.g. by means of an extracorporeal blood circulation device, or an interventional device, for heating and/or cooling the blood flow). Preferably, the hyperthermia treatment system is a full-body hyperthermia treatment system, e.g. configured to raise and maintain the core body temperature at a predetermined value (which is typically elevated with respect to the typical homeostatic temperature of the species).

In a fifth aspect, the present invention relates to a computer-program product for, when executed on a computing device, performing a computer-implemented method 100 in accordance with embodiments of the first aspect of the present invention.

**Claims**

1. A computer-implemented method (100) for predicting and/or simulating a plurality of temperatures in a human or animal body when said body is in a controllable environment, said controllable environment comprising controllable heating and/or cooling means for bringing a core body temperature of said body to a predetermined target temperature, the method comprising:

   - receiving (101) as input at least one property of said body obtained by measurements of the body,
   - calculating (102) at least one body-specific parameter of a bioheat model based on the at least one property of the body,
   - obtaining (103) at least one environment-specific parameter that is indicative of at least one thermal property of said controllable environment and/or of at least one property of a thermal exchange between said body and said controllable environment, and
   - calculating (104) said temperatures in the body by numerically solving said bioheat model taking said body-specific parameters and said environment-specific parameters into account,

   wherein said bioheat model comprises a plurality of equations representative of an equivalent thermal circuit (50) for modeling heat flows in the body and between the body and the controllable environment,
   said equivalent thermal circuit comprising a blood compartment (51) and a plurality of anatomical segments, the plurality of anatomical segments comprising at least a lower extremity segment (52), an upper extremity segment

(53), a trunk segment (54) and a head segment (55),
wherein each of the anatomical segments (52,53,54,55) comprises a plurality of compartments, the plurality of compartments comprising at least one interior compartment (56; 561,562,563) representative of a core and/or bone part of the anatomical segment, at least one intermediate compartment (57; 571,572) representative of a muscle and/or fat part of the anatomical segment and an exterior compartment (58) representative of a skin part of the anatomical segment,
wherein the at least one interior compartment of the at least one trunk segment (54) comprises a plurality of organ compartments (561,562) representative of respectively at least two internal organs,
wherein said at least two organ compartments (561,562) are connected in parallel in said equivalent thermal circuit.

2. The method of claim 1, wherein said obtaining (103) of the at least one environment-specific parameter comprises obtaining a plurality of different values of the or each environment-specific parameter, and performing the step of calculating (104) the temperatures for each of said different values to determine changes in said temperatures with respect to said at least one environment-specific parameter.

3. The method of claim 2, wherein said obtaining (103) of the at least one environment-specific parameter comprises adjusting (105) a previous value of said plurality of different values for which said temperatures were calculated (104) to determine a next value in said plurality of different values for which said temperatures are calculated (104), in order to optimize one or more of said temperatures toward one or more corresponding target temperatures and/or to meet one or more constraints on said temperatures.

4. The method of any of the previous claims, comprising receiving (106), as input, sensor data from one or more temperature sensors on or in the body, and taking said sensor data into account when numerically solving said bioheat model.

5. The method of any of the previous claims, wherein said at least one trunk segment (54) comprises an abdomen segment and a thorax segment, and wherein said plurality of organ compartments (561,562) are interior compartments of the abdomen segment.

6. The method of any of the previous claims, wherein said calculating (102) of the at least one body-specific parameter comprises determining values representative of thermal conduction between at least some of the compartments of said model based on said at least one property of the body.

7. The method of any of the previous claims, wherein said at least one environment-specific parameter comprises:

- at least one value representative of a convection parameter and/or a temperature of an air flow to which the body is exposed in said environment, and/or
- at least one value representative of a temperature of surroundings of the body in said environment for use in calculations of radiative heat transport, and/or
- at least one value indicative of a temperature of an actively heated and/or cooled surface contacting at least part of the body in said environment, and/or
- at least one value indicative of a temperature and/or a humidity of air inspired by the body in said environment, and/or
- at least one value indicative of a controllable thermal property of a heater and/or cooler for heating and/or cooling blood of the body in said environment.

8. The method of claim 7, further comprising:

- calculating a value representative of a rate of convective heat exchange for each exterior compartment (58) based on the at least one value representative of the convection parameter and/or the temperature of the air flow to which the body is exposed in said environment, and/or
- calculating a value representative of a rate of radiative heat exchange for each exterior compartment (58) based on the at least one value representative of the temperature of the surroundings in the environment, and/or
- calculating a value representative of a rate of conductive heat exchange for an exterior compartment (58) based on the at least one value indicative of the temperature of the actively heated and/or cooled surface, and/or
- calculating a value representative of a rate of heat exchange by respiration based on the at least one value indicative of the temperature and/or the humidity of the inspired air.

9. The method of claim 8, wherein calculating any of the values representative of the rate of convective and/or radiative and/or conductive heat exchange takes a surface area into account, said surface area being obtained or calculated from the at least one property of the body received (101) as input.

10. The method of any of the previous claims, wherein said at least one interior compartment of said trunk segment (54) that comprises the at least two organ compartments (561,562) also comprises a remainder compartment (563) representative of the remainder of the interior part of said trunk segment, said remainder compartment (563) and said at least two organ compartments (561,562) being connected in parallel in said equivalent thermal circuit (50).

11. A method (200) comprising: measuring (201) at least one property of a human or animal body, providing (202) the at least one property of the body as input to a computer-implemented method (100) in accordance with any of the previous claims, and performing the computer-implemented method (100).

12. A device (80) for predicting and/or simulating a plurality of temperatures in a human or animal body when said body is in a controllable environment, said controllable environment comprising controllable heating and/or cooling means for bringing a core body temperature of said body to a predetermined target temperature, the device (80) comprising:

   - an input (81) for receiving at least one property of said body obtained by measurements of the body, and
   - a processor (82),

   wherein said processor is adapted for:

   - calculating at least one body-specific parameter of a bioheat model based on the at least one property of the body,
   - obtaining at least one environment-specific parameter that is indicative of at least one thermal property of said controllable environment and/or of at least one property of a thermal exchange between said body and said controllable environment, and
   - calculating said temperatures in the body by numerically solving said bioheat model taking said body-specific parameters and said environment-specific parameters into account,

   wherein said bioheat model comprises a plurality of equations representative of an equivalent thermal circuit (50) for modeling heat flows in the body and between the body and the controllable environment,
   said equivalent thermal circuit comprising a blood compartment (51) and a plurality of anatomical segments, the plurality of anatomical segments comprising at least a lower extremity segment (52), an upper extremity segment (53), a trunk segment (54) and a head segment (55),
   wherein each of the anatomical segments (52,53,54,55) comprises a plurality of compartments, the plurality of compartments comprising at least one interior compartment (56; 561,562,563) representative of a core and/or bone part of the anatomical segment, at least one intermediate compartment (57; 571,572) representative of a muscle and/or fat part of the anatomical segment and an exterior compartment (58) representative of a skin part of the anatomical segment,
   wherein the at least one interior compartment of the at least one trunk segment (54) comprises a plurality of organ compartments (561,562) representative of respectively at least two internal organs,
   wherein said at least two organ compartments (561,562) are connected in parallel in said equivalent thermal circuit.

13. A hyperthermia treatment planning workstation, comprising a device in accordance with claim 12.

14. A hyperthermia treatment system, comprising a device in accordance with claim 12, a controllable environment comprising controllable heating and/or cooling means for bringing a core body temperature of said animal or human body to a predetermined target temperature, and a plurality of temperature sensors for measuring temperatures on or in the body,
   wherein said device is adapted for receiving sensor data from said plurality of temperature sensors, taking said sensor data into account for calculating said temperatures in the body, and controlling said controllable heating and/or cooling means in response to said calculated temperatures in the body.

15. A computer-program product for, when executed on a computing device, performing a method in accordance with any of the claims 1 to 10.

FIG 1

FIG 2

FIG 3

62  61  64  61

52,
53,
55

56

571  572

57

58

63

61

**FIG 4**

80

81  82  83

**FIG 5**

Europäisches
Patentamt

European
Patent Office

Office européen
des brevets

## EUROPEAN SEARCH REPORT

Application Number

EP 19 18 0212

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | XIANG S H ET AL: "Comprehensive evaluation on the heating capacities of four typical whole body hyperthermia strategies via compartmental model", INTERNATIONAL JOURNAL OF HEAT AND MASS TRANSFER, PERGAMON PRESS, GB, vol. 51, no. 23-24, 1 November 2008 (2008-11-01), pages 5486-5496, XP025496477, ISSN: 0017-9310, DOI: 10.1016/J.IJHEATMASSTRANSFER.2008.04.024 [retrieved on 2008-06-02] * whole document, in particular as abstract, paragraph "compartmental model" on page 5487 ; Figures 2, 3, 6-8, 11; Tables 1 and 2; page 5487, right column, last paragraph-page 5491, left column, 1st paragraph; page 5488, left column; page 5492, left column, paragraph 1; equations 25-32 on page 5492-5493) * | 1-15 | INV. G16H20/30 G16H50/30 |
| | ----- | | |
| A | WO 2011/091847 A1 (BRAINLAB AG [DE]; INMACULADA RODRIGUEZ-PONCE MARIA [DE] ET AL.) 4 August 2011 (2011-08-04) * the whole document * | 1-15 | TECHNICAL FIELDS SEARCHED (IPC) G16H |
| | ----- | | |

The present search report has been drawn up for all claims

| Place of search | Date of completion of the search | Examiner |
|---|---|---|
| Munich | 18 December 2019 | Vanmontfort, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................................
& : member of the same patent family, corresponding
document

EPO FORM 1503 03.82 (P04C01)

## EP 3 751 577 A1

**ANNEX TO THE EUROPEAN SEARCH REPORT
ON EUROPEAN PATENT APPLICATION NO.**

EP 19 18 0212

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2019

| Patent document cited in search report | Publication date | Patent family member(s) | Publication date |
|---|---|---|---|
| WO 2011091847 A1 | 04-08-2011 | EP 2528493 A1<br>US 2013035921 A1<br>US 2019167180 A1<br>WO 2011091847 A1 | 05-12-2012<br>07-02-2013<br>06-06-2019<br>04-08-2011 |

EPO FORM P0459

For more details about this annex : see Official Journal of the European Patent Office, No. 12/82

22

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102012973 A

**Non-patent literature cited in the description**

- **PAULIDES et al.** Simulation techniques in hyperthermia treatment planning. *International journal of hyperthermia,* vol. 29 (4), 346-357
- **XIANG et al.** Comprehensive evaluation on the heating capacities of four typical whole body hyperthermia strategies via compartmental model. *International Journal of Heat and Mass Transfer,* vol. 51 (23-34), 5486-5496
- **COCCARELLI et al.** An advanced computational bioheat transfer model for a human body with an embedded systemic circulation. *Biomechanics and Modeling in Mechanobiology,* vol. 15 (5), 1173-1190